# EUROPEAN PATENT APPLICATION

(11) **EP 3 009 142 A1**
(43) Date of publication of application: **20.04.2016**
(21) Application number: 15188991.2
(22) Date of filing: 26.05.2009
(51) Int. Cl.: A61K 38/08, A61K 38/55, A61P 25/04

(54) **OPIORPHIN FOR USE AS ANALGESIC AGENT**

(30) Priority: 26.05.2008 FR 0853400
(62) Divisional of application: 09761603.1
(71) Applicant: Institut Pasteur, 75724 Paris Cedex 15 (FR)
(72) Inventor: ROUGEOT, Catherine, 78460 Chevreuse (FR)
(74) Representative: Blot, Philippe Robert Emile

(57) **Abstract**

The present invention relates to peptides derived from human Basic Proline-rich Lacrimal Protein (BPLP), notably opiorphin, for use as analgesic agents for chronic treatment of pain

## Description

The present invention relates to peptides derived from human BPLP (Basic Proline-rich Lacrimal Protein) protein, notably opiorphin, for use as analgesic agents for chronic treatment of pain.

Psychostimulant agents are psychotropic substances considered as psychic stimulants, which accelerate the activity of the nervous system and stimulate the motivation and well-being. According to the classification of psychotropic agents established by Delay and Deniker, psychostimulant agents notably comprise nooanaleptics, such as the stimulants of alertness (amphetamines), antidepressant thymoanaleptics, as well as various stimulants such as khat and caffeine.

Psychostimulants act by stimulating neurotransmission. They are used for treated various symptoms including vigilance drop, narcolepsy, obesity, attention deficit and/or hyperactivity in children, obsessive-compulsive disorders (OCD), depression, mania and bipolar disease.

However, psychostimulants are often associated with adverse effects such as dependency, tolerance, depression after sudden withdrawal and anxiety. There therefore exists a need for psychostimulants which have limited and/or minimized adverse effects.

A gene regulated by androgens, mainly expressed in the submandibular gland and the prostate of adult rats, was identified in 1988 (Rosinski-Chupin et al. Proc. Natl. Acad. Sci. USA 1988; 85(22):8553-7; EP 0 394 424). This gene codes for a precursor, the "submandibular rat₁ protein" (SMR1). This precursor is cleaved *in vivo* at multibasic sites, giving rise to three structurally close peptides (Rougeot et al., Eur. J. Biochem. 1994; 219(3):765-73).

It was established that the peptide derived from SMR1 of sequence QHNPR (SEQ ID NO: 6), called sialorphin, is a hormonal messenger of intercellular communication. Therefore, sialorphin is an exocrine and endocrine hormonal peptide, the expression of which is regulated by androgens and the secretion caused under an environmental stress mediated by adrenergic signals (Rougeot et al., Am. J. Physiol. 1997; 273(4 Pt2):R1309-20).

The fact that sialorphin is secreted in response to an environmental stress in adult male rats led to the hypothesis that this peptide may play a role in the integration of physiological and behavioral signals related to reproduction. The role played by sialorphin in sexual behavior was evaluated, and experimental data have shown that sialorphin has the capability of modulating the sexual behavior of male rats depending on the environmental background. International Patent Application WO 01/00221 describes the use of maturation products of SMR1 for treating mental and/or behavioral disorders, including sexual disorders.

Further, it was discovered that the SMR1 maturation products recognize specific sites in the organs, said sites being involved in the concentration of minerals. International Patent Application WO 98/37100 describes the therapeutic use of SMR1 maturation products for treating or preventing diseases associated with a disequilibrium of minerals.

Rougeot et al., Proc. Natl. Aca. Sci. USA 2003; 100(14):8549-54) have demonstrated that the surface receptor to which sialorphin binds *in vivo* is the NEP endopeptidase (Neprilysin; EC 3.4.24.11). Further, it was proved that sialorphin is a ligand and a physiological antagonist of NEP activity. Therefore, sialorphin is the first physiological inhibitor of NEP enkephalinase to have been identified in mammals (European Patent Application EP 1 216 707) and that displayed a potent analgesic effect in rat models of pain.

European Patent Application EP 1 577 320 describes the human functional homolog of sialorphin. This peptide, of sequence SEQ ID NO: 2, is called opiorphin. Genomic analyses *in silico* have revealed that it corresponds to a fragment of maturation of the BPLP protein of sequence SEQ ID NO: 5. The BPLP protein is also called "Basic Proline-rich Lacrimal Protein", "Proline-rich protein 1" or "PRL1" and is coded by the human gene *Prol1.* It was shown that opiorphin inhibits degradation of the physiological NEP and APN substrates, i.e. the substance P and the methionine enkephalin. European Patent Application EP 1 577 320 indicates that opiorphin has analgesic properties and that it may notably be used for treating or preventing pain.

The analgesic effect of opiorphin was confirmed subsequently by Rougeot and Messaoudi (Med. Sci. (Paris) 2007; 23(1):37-9). It was also shown that this analgesic effect is not accompanied by an anti-peristaltic effect, unlike what occurs with morphine which is the most powerful analgesic used up to now (Rougeot C, Proceedings of the 4th International Peptide Symposium; J. Wilce (Editor) on behalf of the Australian Peptide Society, 2007).

The inventors have found that, surprisingly, opiorphin not only has analgesic properties, but also a psychostimulant effect. Further, this psychostimulant effect is not associated with any adverse effect of the amnesia, sedation, hyperactivity or addiction type. Finally, it was found that the analgesic potency of opiorphin is as powerful as that of morphine and that its psychostimulant potency is as powerful as that of imipramine.

Therefore, opiorphin and derived peptides may advantageously be used as psychostimulants for treating or preventing diseases such as narcolepsy, hypersomnia, vigilance drop, attention deficit in adults and in children, hyperactivity in adults and in children, attention-deficit/hyperactivity disorder (ADHD), obsessive-compulsive disorders (OCD), and mood disorders such as depression, bipolar disease, dysthymic disorder and cyclothymic disorder.

### Peptides according to the invention

The invention relates to therapeutic applications of a peptide which comprises or consists in a product of maturation of Basic Proline-rich Lacrimal Protein (BPLP) of sequence SEQ ID NO: 5, called a reference peptide or sequence, or a derivative of said maturation product. Such peptides are designated as "peptides according to the invention" in the text of this application.

Within the scope of this invention, by *"peptide"* is meant a molecule comprising a linear chain of amino acids bound to each other by peptide bonds. The chain may possibly be cyclic, i.e. both ends of the linear peptide or side groups of amino acids are bound by a chemical bond. The peptides according to the invention have less than 100 amino acids. Preferably, the peptide consists in 4-40, 4-35, 4-30, 4-20, 4-10, 5-40, 5-35, 5-30, 5-20, 5-10, 5-8 or 5-6 amino acids.

By *"maturation product"* is meant a peptide obtained by selective proteolytic cleavage of the precursor protein, at maturation consensus sites, by a prohormone convertase. Prohormone convertases convert an inactive precursor into active peptides and include, e.g., furin, PC convertases or PACE 4. The sequence of the maturation consensus sites preferably follows the following consensus: [H/R/K]-X₃-[R/K]-[R/K] wherein [H/R/K] means that the amino acid is H, R or K, X₃ designates a chain of three amino acids, and [R/K] means that the amino acid is R or K. Prohormone convertases cleave the consensus unit between dibasic residues [R/K] - [R/K]. Prohormone convertases are well known to one skilled in the art and are notably described by Scamuffa et al. (2006 FASEB J. 20(12):1954-63).

One of the maturation products of the Basic Proline-rich Lacrimal Protein (BPLP) of sequence SEQ ID NO: 5 is the QRFSR peptide of sequence SEQ ID NO: 2. The sequence of the pre-pro-protein BPLP contains a nick consensus site of the signal peptide, the cleavage of which generates the pro-protein QRFSRRX₍ₙ₎. This pro-protein QRFSRRX₍ₙ₎ contains a dibasic site RR and a basic amino acid in position -4 of the nick site between the 2Rs. The QRFSR peptide is generated after action of a prohormone convertase.

The peptides according to the invention also include derivatives of peptides comprising or consisting in maturation products of the Basic Proline-rich Lacrimal Protein (BPLP) of sequence SEQ ID NO: 5.

By *"peptide derivatives"* are meant peptides having an amino acid sequence comprising one or more mutations (substitutions, insertions or deletions) relatively to the reference peptides. Preferably, said peptides only comprise mutations of the substitution type. The substitutions may be conservative or non-conservative. Preferably, said peptides comprise at most 1, 2, 3, 4 or 5 mutations or substitutions relatively to the reference peptides. By *"peptide derivatives"* are meant also peptidomimetics of peptides comprising or consisting in maturation products of the Basic Proline-rich Lacrimal Protein (BPLP) of sequence SEQ ID NO: 5 (see e.g. Abell, 1997, Advances in Amino Acid Mimetics and Peptidomimetics, London: JAI Press ; Gante, 1994, Peptidmimetica, massgeschneiderte Enzyminhibitoren Angew. Chem. 106: 1780-1802 ; and Olson et al., 1993, J. Med. Chem. 36: 3039-3049). Preferred peptidomimetics in accordance with the invention include those described in US provisional application US 61/042922 filed on April 7th 2008 and in international application PCT/EP2009/054171 filed on April 7th 2009.

In a preferred embodiment, the peptide according to the invention differs from the reference sequence only by the presence of conservative substitutions. Conservative substitutions are substitutions of amino acids of the same class, such as substitutions of amino acids on the non-charged side chains (such as asparagine, glutamine, serine, cysteine, tyrosine), of amino acids on basic side chains (such as lysine, arginine and histidine), of amino acids on acid side chains (such as aspartic acid and glutamic acid), of amino acids on apolar side chains (such as alanine, valine, leucine, isoleucine, proline, phenylalanine, and tryptophan).

Preferably, the peptide according to the invention is an isolated peptide. By *"isolated'* peptide is meant here a peptide isolated from the human body or from the organism of a non-human mammal, preferably in purified form. However, the isolated peptide may for example be present in a pharmaceutical composition or in a kit. Preferably, the peptide is present in one of the pharmaceutical compositions described below.

The peptides according to the invention exert a psychostimulant activity. By *"peptide exerting a psychostimulant activity"* is meant a peptide which:
- exerts a protective effect against resignation in a behavioral despair test (for example the forced swimming test in rats); and/or
- inhibits the activity of metallo-ectopeptidases such as NEP (Neprilysin; Neutral endopeptidase; EC 3.4.24.11) and/or APN (Aminopeptidase N; EC 3.4.11.2). Preferably, the peptide inhibits both NEP and APN activity. Without being limited by a particular theory, the inventors believe that the peptides according to the invention by inhibiting degradation of enkephalins by these two metallo-ectopeptidases, potentialize their physiological action in terms of amplitude of action and of duration of action and thereby activates the opioid pathways, more particularly the enkephalin-dependent µ- and δ-opiod receptors.

The protective effect against resignation may be determined by the forced swimming test in rats. In this test, when a peptide exerts a protective effect against resignation, the immobility time of rats to which said peptide has been administered is reduced relatively to the immobility time of control rats. This test comprises the following tests:
- a pre-test session, during which the rat is deposited in water for a determined time (between 10 and 20 min, for example 15 min), and then removed from the water, dried and put back in its cage.
- a few hours later (between 20 and 25 hours later, for example 24 hours later), a test session during which the rat is again deposited in the water for a determined time (between 5 and 8 min, for example 5 min), and then removed from the water, dried and put back in its cage.

The peptide to be tested is administered after the pre-test session and before the test. The immobility time of the rats is recorded for 5 min during the pre-test and the test. The immobility time of the rats to which the peptide has been administered is compared with that of control rats and with that of the same rats during the pre-test session. Example 3 describes in more detail the protocol of the forced swimming test in rats.

Inhibition of the activity of metallo-ectopeptidases such as NEP and/or APN may be measured by any technique well known to one skilled in the art. One of the methods described in Example 2 of European Patent Application EP 1 577 320 may for example be used. For example, it is possible to measure the inhibitory activity of NEP by measuring the inhibition of degradation of the substance P in preparations of membranes of LNCaP cells. This protocol is described in detail in Rougeot et al. (Proc. Natl. Acad. Sci. USA 2003; 100(14):8549-54) and in international application PCT/EP2009/050567 filed on January 19, 2009.

In a particular embodiment, the peptides according to the invention comprise or consist in a fragment of the sequence SEQ ID NO: 5 or comprise or consist in a peptide derived from said fragment of the sequence SEQ ID NO: 5.

Preferably, the peptides according to the invention comprise or consist in a sequence X₁-X₂-Arg-Phe-Ser-Arg (SEQ ID NO: 1), wherein:
- X₁ represents a hydrogen atom, a tyrosine or a cysteine;
- when X₁ is a hydrogen atom, X₂ represents a glutamine or a pyroglutamate;
- when X₁ is a tyrosine or a cysteine, X₂ represents a glutamine; and
- said sequence X₁-X₂-Arg-Phe-Ser-Arg is the C-terminal end of said peptide.

Preferably, the peptide according to the invention consists in the sequence X₁-X₂-Arg-Phe-Ser-Arg (SEQ ID NO: 1). The peptide according to the invention may for example consist in the QRFSR sequence (SEQ ID NO: 2), YQRFSR (SEQ ID NO: 3), CQRFSR (SEQ ID NO: 4) or GlpRFSR. Alternatively, the peptide according to the invention may differ from the sequences SEQ ID NO: 2, SEQ ID NO: 3 or SEQ ID NO: 4 by conservative substitutions.

In another particular embodiment, the peptides according to the invention comprise or consist in maturation products of an allelic variant of the Basic Proline-rich Lacrimal Protein (BPLP) of sequence SEQ ID NO: 5.

For the sake of clarity, it is specified that sialorphin of sequence SEQ ID NO: 6 is excluded from the peptides according to the invention.

The peptide according to the invention may further include one or more chemical modifications improving its stability and/or its bioavailability. This modification may for example be aimed at obtaining a more stable and more lipophilic peptide than the initial peptide. The peptides including one or more chemical modifications improving their stability and/or their bioavailability are part of the peptides according to the invention.

Such chemical or enzymatic modifications are well known to one skilled in the art. In a non-limiting way, mention may for example be made of the following modifications:
- modifications of the C-terminal or N-terminal end of the peptides such as N-terminal deamination or acylation (preferably acetylation), or such as C-terminal amidation or esterification;
- modifications of the amide bond between two amino acids, such as acylation (preferably acetylation) or alkylation at the alpha nitrogen or carbon;
- changes in chirality, such as the substitution of a natural amino acid (L-enantiomer) by the corresponding D-enantiomer. This modification may possibly be accompanied by an inversion of the side chain (from the C-terminal end to the N-terminal end);
- changes into azapeptides, in which one or more alpha carbons are replaced with nitrogen atoms; and/or
- changes into betapeptides, in which one or more carbons are added on the N-alpha side or on the C-alpha side of the main chain.

As such, one or more of the amino acids, serine (Ser) and/or threonine (Thr), of the peptides may be modified, notably by introducing at the OH group of the side chain, serine and/or threonine, an ester group, an ether group or an C₈ octanoyl group. Esterifaction, a simple operation, may be carried out with a carboxylic acid, an anhydride, by bridges, etc., in order to form acetates or benzoates for example. Etherification, which gives more stable compounds, may be carried out with an alcohol, a halide, etc., in order to form a methylether or an O-glycoside for example.

Moreover one or more amino acids, lysine (Lys), of the peptides may also or alternatively be modified, notably by:
- amidation: this modification is simple to accomplish, the positive charge of lysine being substituted with hydrophobic groups (for example acetyl or phenylacetyl);
- amination: by forming secondary amides from the primary amine R= (CH₂)₄-NH₃⁺, for example by forming N-methyl, N-allyl or N-benzyl groups; and
- by forming N-oxide, N-nitroso, N-dialkyl phosphoryl, N-sulfenyl, or N-glycoside groups.

Moreover one or more amino acids, glutamine (Gin), may also or alternatively be modified for example by amidation, by forming secondary or tertiary amines, notably with groups of the methyl, ethyl type, either functionalized or not.

Moreover, one or more amino acids, glutamate (Glu) and/or aspartate (Asp), may also or alternatively be modified, for example:
- by esterification, in order to form methyl esters either substituted or not, ethyl esters, benzyl esters, thiols (activated esters); and
- by amidation, notably in order to form N,N-dimethyl, nitroanilide, pyrrolidinyl groups.

On the other hand, it is preferable not to modify the proline amino acids, which participate in the secondary structure of the peptides, further being aware that the amino acids, Gly, Ala and Met, do not generally provide modification possibilities which would obviously be of interest.

Examples of such peptides including one or more chemical modifications improving their stability and/or their bioavailability are described in US provisional application US 61/042922 filed on April 7th 2008 and in international application PCT/EP2009/054171 filed on April 7th 2009. These include peptides of the following formula (I):

ζ -AA₁-AA₂- AA₃-AA₄-AA₅-OH (I),

wherein:
- ζ is hydrogen atom, tyrosine, Y-[linker]- or a Zn chelating group, such as cysteine, C-[linker]-, N-acetyl-cysteine, N-mercaptoacetyl (HS-CH₂-CO-), hydroxamic acid (HO-NH-CO-) or an optionally substituted hydroxyquinoline,
- AA₁ is Q or Glp,
- AA₂ is K, R or H, preferably R,
- AA₃ is Y, G, N, F or F(X), preferably F or F(X),
- AA₄ is P, S or S(OAlk), preferably S or S(OAlk),
- AA₅ is K or R, preferably R,
- C-[linker]- meaning Cys-[NH-(CH₂)n-CO]-, wherein n is an integer between 1 and 20,
- Y-[linker]- meaning Tyr-[NH-(CH₂)_{n'}-CO]-, wherein n' is an integer between 1 and 20,
- F(X) meaning a phenylalanine, the phenyl group of which is substituted by one or more halogen atoms,
- S(OAlk) meaning a serine, the hydroxyl group of which is substituted by a linear or branched alkyl group having from 1 to 20 carbon atoms,
- said AA₁, AA₂, AA₃, AA₄, and AA₅ may be independently either in the L-configuration or D-configuration, and any one of AA₁, AA₂, AA₃, AA₄, and AA₅ may be optionally a b aminoacid, an aza-aminoacid or a b-aza-aminoacid;
wherein if the peptide derivative comprises a cysteine, said peptide derivative is optionally a dimer, with the proviso that the peptide is not QRFSR, QHNPR, QRGPR, YQRFSR or GlpRFSR.

Among the peptides of formula (I), preferred peptides include those inhibiting both NEP and APN. Such peptides may have the following formula (IV):

ζ ^{m}-Q-R-AA^{m}₃-AA^{m}₄-R-OH (IV),

wherein:
- ζ^{m} is a hydrogen atom, or a Zn chelating group, such as cysteine, C-[linker]-, N-acetyl-cysteine, N-mercaptoacetyl (HS-CH₂-CO-), hydroxamic acid (HO-NH-CO-) or an optionally substituted hydroxyquinoline,
- AAⁿ₃ is F or F(X), preferably F(X),
- AA^{m}₄ is S or S(OAlk), preferably S or S(OAlk),
- C-[linker]-, F(X) and S(OAlk) being as defined hereabove for peptides of formula (I),
- said Q, R, AA₃, AA₄, and R may be independently either in the L-configuration or D-configuration ,and any one of Q, R, AA₃, AA₄, and R may be optionally a β aminoacid, an aza-aminoacid or a β-aza-aminoacid;
wherein if the peptide derivative comprises a cysteine, said peptide derivative is optionally a dimer, with the proviso that the peptide is not QRFSR or YQRFSR.

Specific examples of such peptide of formula (I) and/or (IV) include:
- QRFSR-NH₂;
- QR-F[4Br]-SR, wherein -F[4Br]- is a phenylalanine, the phenyl group of which is substituted in the para position by a bromo atom;
- QRFPR;
- (Acetyl)-QRFSR;
- C-(-HN-(CH₂)₈-CO-)-QRFSR;
- biotine-(-HN-(CH₂)₆-CO-)-QRFSR;
- dR-dS-dF-dR-dQ;
- Y-(-HN-(CH₂)₆-CO-)-QRFSR;
- Y-(-HN-(CH₂)₁₂-CO-)-QRFSR;
- QRF-S(O-octanoyl)-R;
- CQRFSR;
- CQRF-S(O-octanoyl)-R;
- CQRF-S(O-dodecanoyl)-R;
- C-(-HN-(CH₂)₈-CO-)-QRFSR;
- C-(-HN-(CH₂)₁₂-CO-)-QRFSR;
- C-(-HN-(CH₂)₈-CO-)-QRF-S(O-octanoyl)-R;
- [Cß2]QRF-S(O-octanoyl)-R;
- C-(-HN-(CH₂)₈-CO-)-QRFS-[ß3R];
- C-[dQ]-RF-S(O-octanoyl)-[dR];
- C-(-HN-(CH₂)₈-CO-)-QRFS-[dR];
- [dC]-QRF-S(O-octanoyl)-[dR];
- [Cß2]-QRF-S(O-octanoyl)-[ß3R];
- [CQRFSR]₂;
- QRYSR;
- QR-F[4F]-SR, wherein -F[4F]- is a phenylalanine, the phenyl group of which is substituted in the para position by a fluoro atom;
- QR-F[4Br]-SR, wherein -F[4Br]- is a phenylalanine, the phenyl group of which is substituted in the para position by a bromo atom;
- QKFSR;
- QRFSK;
- C-(-HN-(CH₂)₆-CO-)-QRFSR;
- C-(-HN-(CH₂)₆-CO-)-QRF-S(O-octanoyl)-R;
- C(-HN-(CH₂)₁₂-CO-)QRF-S(O-octanoyl)-R;
- C-(-HN-(CH₂)₁₂-CO-)-QRFS-dR;
- C-(-HN-(CH₂)₁₂-CO-)-QRF-S(O-octanoyl)-β3R;
- C-(-HN-(CH₂)₈-CO-)-QRF-S(O-octanoyl)β3R;
wherein :
- Cß2 is H₂N(-CH₂-SH)-CH₂-CO-;
- ß3R is -NH-CH₂-C[-(CH₂)₃-NH-C(NH)(NH₂)]-COOH;
- -S(-O-octanoyl) means a serine, the hydroxyl group of which is substituted by an octanoyl group,
- -S(-O-dodecanoyl) means a serine, the hydroxyl group of which is substituted by a dodecanoyl group.

The peptides including one or more chemical modifications improving their stability and/or their bioavailability that are described in US provisional application US 61/042922 filed on April 7th 2008 and in international application PCT/EP2009/054171 filed on April 7th 2009 further include:
- NH2-QRFSR-CONH2;
- NH2-QRGPR-COOH;
- NH2-QHNPR-COOH;
- NH2-QR(4BromoF)SR-COOH;
- NH2-QRFPR-COOH;
- N-(acetyl)QRFSR-COOH;
- N-(C8-polyethylene)QRFSR-COOH;
- N-(biotin-C6)QRFSR-COOH;
- NH2-dRdSdFdRdQ-COOH(D-enantiomer retroinversion);
- NH2-YQRFSR-COOH;
- NH2-Y-(C6-polyethylene)QRFSR-COOH;
- NH2-Y-(C12-polyethylene)QRFSR-COOH;
- NH2-QRF[S-O-C8-polyethylene]R-COOH;
- NH2-CQRFSR-COOH;
- NH2-CQRF[S-O-C8-polyethylene]R-COOH;
- NH2-CQRF[S-O-C12-polyethylene]R-COOH;
- NH2-C-(C8-polyethylene)QRFSR-COOH;
- NH2-C-(C12-polyethylene)QRFSR-COOH;
- NH2-C-(C8-polyethylene)QRF[S-O-C8-polyethylene]R-COOH;
- NH2-[Cβ2]QRF[S-O-C8-polyethylene]R-COOH;
- NH2-C-(C8-polyethylene)QRFS[β3R]-COOH;
- NH2-C[dQ]RF[S-O-C8-polyethylene][dR]-COOH;
- NH2-C-(C8-polyethylene)QRFS[dR];
- NH2-[dC]QRF[S-O-C8-polyethylene][dR]-COOH;
- NH2-[Cβ2]QRF[S-O-C8-polyethylene][β3R]-COOH; and
- [CQRFSR]2, i.e. a dipeptide formed after oxidation of the SH groups of the N-terminal cysteine and cystine bond (disulfide bridge);
wherein:
- Cβ2 replaces the natural cysteine residue with a cysteine-β2 comprising a methyl residue adjacent to the carbon beside the carbonyl group of the cysteine;
- β3R replaces the natural arginine residue with an arginine-β3 comprising a methyl residue adjacent to the carbon beside the amine group of arginine;
- [S-O-C8-polyethylene] is [S-O-octanoyl]; and
- C6, C8 or C12 polyethylenes correspond to a spacer group consisting of an ethylene chain of 6, 8 or 12 carbons.

When the peptide contains but does not consist in a maturation product of the Basic Proline-rich Lacrimal Protein (BPLP) of sequence SEQ ID NO: 5, or contains but does not consist in a derivative of said maturation product, the peptide contains additional amino acids at its N- or C-terminal end. These additional amino acids may for example correspond to a portion of the BPLP sequence, or a sequence of amino acids improving its stability and/or its bioavailability. However, the size of the peptide should preferably not exceed 30-40 amino acids.

The peptides according to the invention may be synthesized by any method well known to one skilled in the art. Such methods notably include conventional chemical synthesis (in a solid phase or in a homogenous liquid phase), enzymatic synthesis from constitutive amino acids or their derivatives, as well as biological production methods by recombinant host cells. Synthesis via a chemical route is particularly advantageous for reasons of purity, antigenic specificity, absence of undesired secondary products and for its ease of production. Synthesis via a chemical route includes among other methods, the Merrifield type synthesis and the methodology of peptide synthesis in a Fmoc solid phase (see for example "Fmoc solid phase peptide synthesis, a practical approach", published by W.C. Chan and P.D. White, Oxford University Press, 2000).

### Therapeutic use of the peptides according to the invention

The invention relates to the peptides according to the invention, described in the above paragraph, for a use as analgesics for chronic treatment of pain.

It has been shown that opiorphin exerts both an antidepressant effect and a psychostimulant effect in a model of analysis of behavioral despair in rats. Further, it has been shown that the antidepressant effect and the psychostimulant effect of opiorphin are dependent on the activation of the endogenous µ- and δ-opioid receptors, but not on activation of endogenous κ-opioid receptors.

Therefore, the application describes the peptides according to the invention, described in the above paragraph, for a use as psychostimulants. Such peptides according to the invention may be used for activating an opioidergic pathway dependent on µ- and/or δ-opioid receptors. Moreover, the peptides according to the invention do not activate opioidergic pathways depending on κ-opioid receptors.

These peptides may notably be used for treating or preventing narcolepsy, hypersomnia, vigilance drop, attention deficit (in adults and in children), hyperactivity (in adults and in children), attention-deficit/hyperactivity disorder (ADHD), obsessive-compulsive disorders (OCD), and mood disorders such as depressive conditions and depression ("Major Depressive Disorder"), the latter including primary depression ("Major Depressive Disorder, Single Episode") and resistant depression ("Major Depressive Disorder, Recurrent"), bipolar disease (of type I and/or of type II), dysthymic disorder and cyclothymic disorder.

Within the scope of treating one of these diseases, the peptides according to the invention are preferably administered to a sub-group of patients needing a psychostimulant and/or activation of an opioidergic pathway depending on µ- of δ-opioid receptors.

The peptides according to the invention may also be used as a combination (i.e. by simultaneous or sequential administration) with a second active ingredient aimed at treating or preventing the same disease. This second active ingredient may also have a psychostimulant effect, or an antidepressant effect, or even have an anxiolytic effect. The peptides may for example be used in combination with at least one second active ingredient selected from:
- an antidepressant such as an imao (iproniazide, moclobemide, etc.) an imipraminic anti-depressant (imipramine, clomipramine, amitriptyline, amoxapine, dosulepin, doxepin, maprotiline, trimipramine, etc.) a selective inhibitory antidepressant of recapture of serotonin (citalopram, fluvoxamine, fluoxetine, paroxetine, sertraline, escitalopram, etc.) or an inhibitory antidepressant of recapture of serotonin and of adrenalin (milnacipran, venlafaxine, mirtazapine, etc.);
- an anxiolytic such as benzodiazepine (alprazolam, clobazam, diazepam, lorazepam, prazepam, etc.), meprobamate, hydroxyzine, buspirone, captodiame or etifoxine; and
- a psychostimulant such as morphine, modafinil, methylphenidate, derivatives of deanol (acti 5, cleregil, debrumyl, etc.), ketoglutarate, adrafinil or sulbutiamine.

Most of these known active ingredients have significant adverse effects at the effective doses. Unlike the known active ingredients, the peptides according to the invention act on a physiological regulation system and not directly on transfer systems or receptors. The action of the peptides according to the invention therefore does not depart from the scope of a regulated system, and cannot saturate the system. Therefore, they have no or very little adverse effects.

The peptides according to the invention, by potentializing the effects of the second active ingredient, allows it to be administered at a concentration of less than the effective dose when the second active ingredient is administered alone. Therefore, a preferred embodiment of the invention relates to the use of peptides according to the invention in combination with at least one second active ingredient, said second active ingredient being administered at a concentration of less than the effective dose, in particular at a dose at which adverse effect is limited, reduced, minimized or abolished.

The peptide according to the invention and the second active ingredient may be present within the same pharmaceutical composition or in two separate pharmaceutical compositions. In the second case, the pharmaceutical compositions may be administered to the patient either essentially simultaneously or sequentially.

The disease mentioned here may be treated at any stage of the disease. By *"treatment"* is meant a curative treatment (directed to at least relieving, slowing down or stopping the progression of the pathology). By *"prevention"* is meant a prophylactic treatment (directed to reducing the risk of occurrence of the pathology).

By *"psychostimulant"* and *"psychostimulant agent"* is meant a compound which causes an increase in dopaminergic and noradrenergic neurotransmission. More particularly, psychostimulants stimulate the neurotransmitters responsible for controlling attention, motivation, alertness and concentration. Dopamine and adrenaline are the two major neurotransmitters responsible for controlling attention, motivation, alertness, concentration.

The term of *"opioidergic pathway depending on µ- and*/*or δ-opioid receptors"* is well known to one skilled in the art. These pathways notably include those described by Henriksen and Willoch (2008 Brain. 131(Pt 5):1171-96). One skilled in the art has several tests available with which it may be evaluated whether a compound activates an opioidergic pathway depending on µ- and/or δ-opioid receptors, such as those described in Example 2. For example, it is possible to compare the effect of the compound in the presence and in the absence of a specific opioid antagonist of µ- and/or δ-opioid receptors. Abolition of the effect of the compound in the presence of the antagonist indicates that the compound activates the investigated opioidergic pathway.

The present invention relates to both the use *in vitro* and use *in vivo* of peptides according to the invention for activating an opioidergic pathway depending on µ- and/or δ-opioid receptors.

The invention also deals with the use of peptides according to the invention for preparing:
- a psychostimulant;
- a drug capable of activating an opioidergic pathway depending on µ- and/or δ-opioid receptors; and/or
- a drug for preventing or treating a disease selected from narcolepsy, hypersomnia, vigilance drop, attention deficit in adults and in children, hyperactivity in adults and in children, attention-deficit/hyperactivity disorder (ADHD), obsessive-compulsive disorders (OCD), and mood disorders such as depression, bipolar disease, dysthymic disorder and cyclothymic disorder.

Moreover, the invention deals with a method for:
- treating or preventing a disease selected in the group consisting of narcolepsy, hypersomnia, vigilance drop, attention deficit in adults and in children, hyperactivity in adults and in children, attention-deficit/hyperactivity disorder (ADHD), obsessive-compulsive disorders (OCD), and mood disorders such as depression, bipolar disease, dysthymic disorder and cyclothymic disorder; and/or
- activating an opioidergic pathway depending on µ- and/or δ-opioid receptors;
comprising the step of administering a peptide according to the invention to an individual. Preferably, the individual is an individual and/or patient in need thereof. Preferably, an effective amount of said peptide is being administered. The individual and/or patient is preferably in need of a psychostimulant and/or of activation of an opioidergic pathway depending on µ- and/or δ-opioid receptors.

### Pharmaceutical compositions and dosage

The peptides according to the invention are preferentially used as a pharmaceutical composition comprising as an active ingredient, at least one peptide according to the invention with one or more pharmaceutically acceptable excipients.

By *"excipient"* or *"pharmaceutically acceptable carrier*", is meant any solvent, dispersion medium, absorption-delaying agent, etc. which does not produce any adverse e.g. allergic reaction in humans or animals.

The peptide according to the invention may correspond to any of the peptides described above.

For example, the pharmaceutical composition according to the invention comprises between 3 and 100, 5 and 50 or 10 and 25 mg of a peptide according to the invention per pharmaceutical composition unit dose. The pharmaceutical composition may be accompanied by instructions relating to the therapeutic applications (for example the ones mentioned above) and the dosage (for example compliant with the dosages mentioned in the previous paragraph).

The dose notably depends on the relevant active ingredient, on the mode of administration, on the therapeutic indication, on the age, weight, and condition of the patient.

The initial dosage is generally selected so as to be as low as possible (minimum effective dose). If necessary, the dosage is gradually increased depending on the response of the patient. Given that there may be a delay before occurrence of the effects of the active ingredient, it will preferably be necessary to wait for several days to a few weeks before increasing the dosage.

In the case of the peptide according to the invention, the initial dose for an adult may for example be less than or equal to 75 mg per day, administered in three times. This dosage may gradually be increased up to 150 mg daily if necessary. Dosages above 200 mg daily are not recommended. However, when patients are sent to hospital in a serious condition, the dosages may reach up to 250 or 300 mg daily. In elderly patients, the initial dose is for example less than or equal to 30 mg daily and preferably it should not exceed 100 mg daily. In children, the initial dose may for example be less than or equal to 10-25 mg daily and preferably should not exceed 75 mg daily.

The pharmaceutical compositions according to the invention may further contain at least one second active ingredient selected from a psychostimulant, an antidepressant and/or an anxiolytic.

The pharmaceutical compositions according to the invention may be formulated so as to be administered to the patient via a single route or via different routes.

The pharmaceutical composition may for example be administered via an oral, sublingual, nasal, buccal, transdermal, intravenous, subcutaneous, intramuscular, and/or rectal route.

In the case of administration via an oral or sublingual route, the compositions of the invention are for example as gelatin capsules, effervescent tablets, naked or coated tablets, sachets, "dragées", drinkable ampoules or solutions, microgranules or prolonged release forms.

In the case of an administration via a nasal or buccal route, the compositions of the invention exist as a spray for example.

In the case of an administration via a transdermal route, the compositions of the invention exist as a patch for example.

When administration via a parenteral route is contemplated, more particularly by injection, the compositions of the invention comprising the active ingredient(s) exist as injectable solutions and suspensions, packaged in ampoules or flasks for slow perfusion. The injection may notably be performed via a subcutaneous, intramuscular or intravenous route. The forms for parenteral administration are conventionally obtained by mixing the active ingredient(s) with buffers, stabilizers, preservatives, solubilizers, isotonic agents and suspension agents. According to known techniques, these mixtures are then sterilized (for example by filtration) and then packaged as intravenous injections.

As a buffer, one skilled in the art may use buffers based on an organic phosphate source.

Examples of suspension agents include methylcellulose, hydroxyethylcellulose, hydroxypropylcellulose, acacia and sodium carboxymethylcellulose.

Further, useful stabilizers according to the invention are sodium sulfite and sodium metasulfite, while mention may be made of sodium p-hydroxybenzoate, sorbic acid, cresol or chlorocresol as preservatives. For preparing an oral solution or suspension, the active ingredients are dissolved or suspended in a suitable vehicle with a dispersant, a humectant, a suspension agent (for example polyvinylpyrrolidone), a preservative (such as methylparaben or propylparaben), a taste-correcting agent or a coloring agent.

For preparing microcapsules, the active ingredients are combined with suitable diluents, suitable stabilizers, agents promoting prolonged release of active substances or any other type of additive for forming a central core which is then coated with a suitable polymer (for example a water-soluble resin or a water-insoluble resin). The techniques known to one skilled in the art will be used for this purpose.

The thereby obtained microcapsules are then optionally formulated in suitable dosage units.

Although having distinct meanings, the terms "comprising", "containing", "including" and "consisting in" have been used interchangeably in the description of the invention and they may be replaced with each other.

The following items are further described:
1. A peptide for a use as a psychostimulant, wherein said peptide:
   - comprises a maturation product of the Basic Proline-rich Lacrimal Protein (BPLP) of sequence SEQ ID NO: 5 or a derivative of said maturation product; and
   - exerts a psychostimulant activity.
2. A peptide for activating an opioidergic pathway depending on µ- and/or δ-opioid receptors, wherein said peptide:
   - comprises a maturation product of the Basic Proline-rich Lacrimal Protein (BPLP) of sequence SEQ ID NO: 5 or a derivative of said maturation product; and
   - exerts a psychostimulant activity.
3. The peptide according to item 1 or 2, wherein said peptide comprises the sequence X₁-X₂-Arg-Phe-Ser-Arg (SEQ ID NO: 1), wherein:
   - X₁ represents a hydrogen atom, a tyrosine or a cysteine;
   - when X₁ is a hydrogen atom, X₂ represents a glutamine or pyroglutamate;
   - when X₁ is a tyrosine or a cysteine, X₂ represents a glutamine; and
   - the sequence of the C-terminal extremity of said peptide consists of X₁-X₂-Arg-Phe-Ser-Arg.
4. The peptide according to item 3, wherein said peptide consists of the sequence X₁-X₂-Arg-Phe-Ser-Arg.
5. The peptide according to any of items 1 to 4, wherein said peptide consists of a sequence selected from the sequences QRFSR (SEQ ID NO: 2), YQRFSR (SEQ ID NO: 3), CQRFSR (SEQ ID NO: 4) and GlpRFSR.
6. The peptide according to any of items 1 to 4, the sequence of which differs from a sequence selected from sequences SEQ ID NO: 2, SEQ ID NO: 3 and SEQ ID NO: 4 by conservative substitutions.
7. The peptide according to any of items 1 to 6, wherein said peptide includes a chemical modification improving its stability and/or bioavailability.
8. The peptide according to any of items 1 to 7, for treating or preventing a disease selected in the group consisting of an obsessive-compulsive disorder (OCD), narcolepsy, hypersomnia, vigilance drop, attention-deficit/hyperactivity disorder (ADHD), attention deficit and/or hyperactivity in adults and in children, depression, bipolar disease, dysthymic disorder and cyclothymic disorder.
9. The peptide according to item 8, wherein said disease is selected from the group consisting of an obsessive-compulsive disorder, narcolepsy, hypersomnia, vigilance drop, attention deficit and/or hyperactivity in children, bipolar disease, dysthymic disorder and cyclothymic disorder.
10. The peptide according to item 9, wherein said disease is selected from the group consisting of an obsessive-compulsive disorder, narcolepsy, hypersomnia and vigilance drop.
11. The peptide according to any of items 1 to 10, intended to be administered to an adult at a dose less than or equal to 75 mg per day.
12. The peptide according to any of items 1 to 10, intended to be administered to a child at a dose less than or equal to 25 mg per day.
13. The peptide according to item 11 or 12, intended to be administered three times daily.
14. The peptide according to any of items 1 to 13, intended to be administered via a route selected from the oral, transdermal, nasal, sublingual and intravenous route.
15. The peptide according to any of items 1 to 14, intended to be administered in combination with a second active ingredient selected from the group consisting of a psychostimulant, an antidepressant and an anxiolytic.
16. A method for treating or preventing a disease selected from the group consisting of an obsessive-compulsive disorder, narcolepsy, hypersomnia, vigilance drop, attention-deficit/hyperactivity disorder, attention deficit and/or hyperactivity in adults and in children, depression, bipolar disease, dysthymic disorder and cyclothymic disorder comprising the step of administering an effective amount of a peptide according to any of items 1 to 15 to an individual in need thereof.

The following examples and figures illustrate the invention without limiting the scope thereof.

### DESCRIPTION OF THE FIGURES

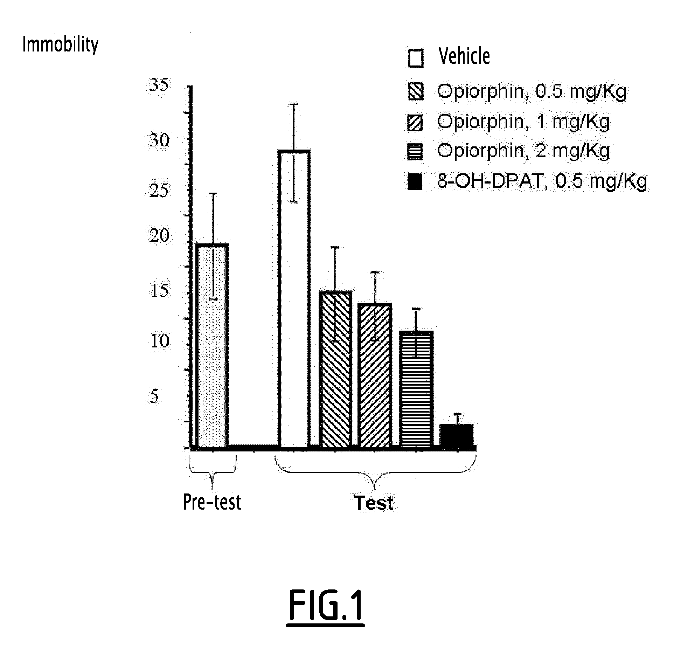

Figure 1 illustrates the psychostimulant and antidepressant effect of opiorphin in the forced swimming test, by measuring the immobility duration during the Pre-Test (n=40) and the Test (dry, n=8 rats/group) (average ± SEM). The results of the Man-Whitney test (vs. vehicle) are as follows: ^{T} p<0.10; * p<0.05; *** p<0.001. During the test, the rats to which opiorphin was administered do not resign themselves, unlike the rats to which the vehicle was administered. This demonstrates that opiorphin has an antidepressant effect. Further, during the test, the immobility time of the rats to which opiorphin was administered is shorter than the immobility time of the same rats during the pre-test. This demonstrates that opiorphin has a psychostimulant effect.

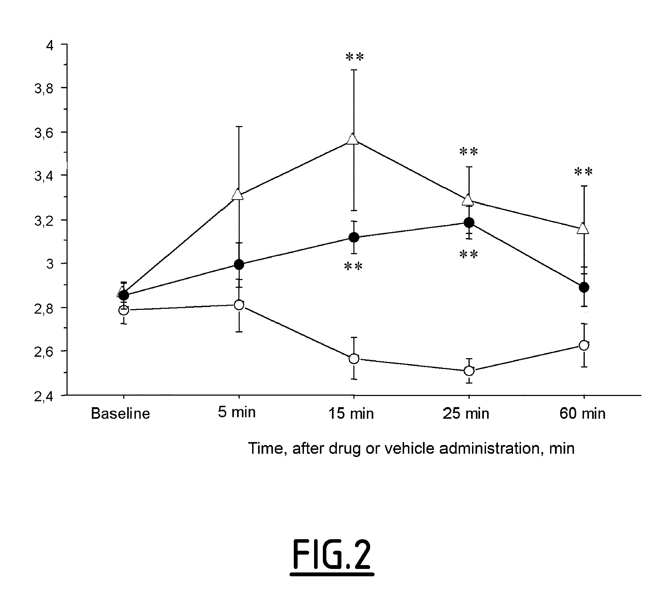

Figure 2 shows that opiorphin exerts an analgesic activity in tail-flick pain model. The pain response was evaluated in function of time to noxious thermal stimuli following administration of human Opiorphin or morphine. Tail-flick latencies were evaluated for 4 different time points: 5, 15, 25 and 60 min after Opiorphin or vehicle administration in reference to morphine. Two consecutive measurements separated by 30 s intervals were carried out before Opiorphin, morphine or vehicle injection to assess for baseline tail-flick latency. Effects of Opiorphin (black circle; 2 mg/kg i.v.) compared to vehicle (open circle) and morphine (open triangle; 1 mg/kg i.v) on the tail-flick latency. Results are expressed as means ± SEM of 6 rats. Asterisk indicates ** P<0.01 vs vehicle by Mann-Whitney U test.

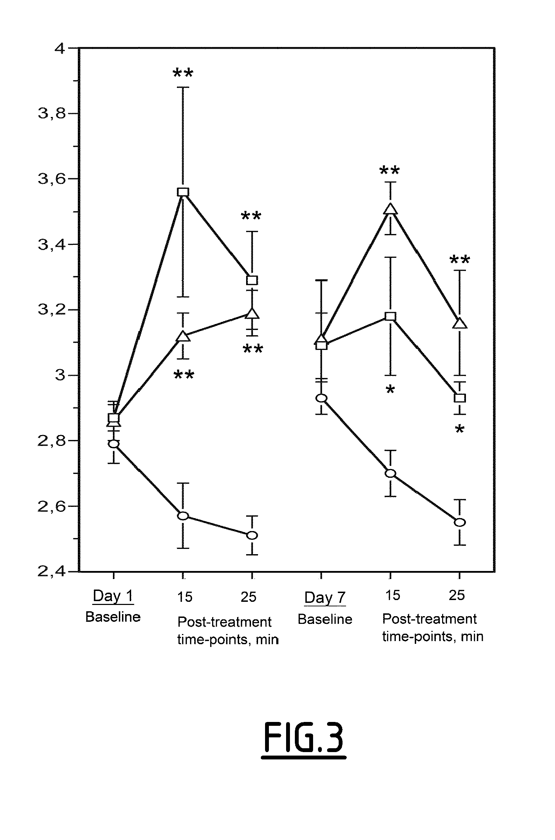

Figure 3 shows that human Opiorphin exhibits an analgesic effect in the tail-flick pain model. The pain response was evaluated in function to noxious thermal stimuli following acute and chronic administration of human Opiorphin or morphine. Tail-flick latencies were evaluated after acute (Day 1) or daily i.v. administration during 7 days (Day 7) of Opiorphin, vehicle or morphine. Two consecutive measurements separated by 30 s intervals were carried out before Opiorphin, morphine or vehicle injection in order to assess baseline tail-flick latency. Effects of Opiorphin (open triangle; 2 mg/kg i.v.) compared to vehicle (open circle) and morphine (open square; 1 mg/kg i.v) on the tail-flick latency. Results are expressed as means ± SEM of 6 rats. Asterisk indicates *P<0.05, ** P<0.01 vs vehicle by Mann-Whitney U test.

### DESCRIPTION OF THE SEQUENCES OF SEQUENCE LISTING

SEQ ID NOs: 1, 2, 3 and 4 correspond to peptides according to the invention. More particularly, SEQ ID NO: 2 corresponds to opiorphin.
SEQ ID NO: 5 corresponds to the sequence of human Basic Proline-rich Lacrimal Protein (BPLP).
SEQ ID NO: 6 corresponds to sialorphin.

### EXAMPLES

### Example 1: Synthesis of opiorphin (i.e. the QRFSR peptide) by Génosphère Biotechnologies (F) & Almac Sciences (UK)

The opiorphin batches were synthesized by Génosphère Biotechnologies (F) & Almac Sciences (UK). It was confirmed that these synthesis batches protect *in vitro,* in a dose-dependent way:
- the substance P (physiological substrate of NEP) and synthetic fluorogenic substrates [Abz]-dRGL-[EDDnp], [Abz]-RGFK-[DnpOH] (Thermo-Fisher Scientific), and from endoproteolysis by recombinant human NEP (inhibitory concentrations of QRFSR peptides, comprised between 5 and 50 µM); and
- the synthetic substrate of Ala-AMC from aminoproteolysis by recombinant human APN.

### Example 2: Effect of opiorphin on nociceptive transmission pathways

It was shown in the analytical model of the acute pain behavioral response in male rats, the "Pin Pain Test" (mechanical stimulus) described in Rougeot et al. (Proc. Natl. Acad. Sci. USA. 2003; 100(14):8549-54), that opiorphin exerts a potent antinociceptive activity at 1 mg/kg i.v. (p= 0.0002, Mann-Whitney U-test, n=8-12 rats/group), equivalent to that of morphine at 6 mg/kg i.p. (Rougeot and Messaoudi, Med. Sci. (Paris). 2007; 23(1):37-9).

In a remarkable way, among the major adverse effects, the anti-peristaltic effect of morphine (80-90% inhibition of peristaltism in rats treated with morphine as compared with control rats) is not observed in rats treated with opiorphin.

An analgesic action of opiorphin was then investigated in the analytical model of chronic pain behavioral response related to subcutaneous chronic inflammation (a chemical stimulus at the hind paw), the "formalin test" described in Rougeot et al. (Proc. Natl. Acad. Sci. USA. 2003; 100(14):8549-54). The reference substance is morphine, at a dose of 2 mg/kg.

On the basis of the two most important variables in the so-called "formalin" pain test, the duration of licking of the paw and the number of spasms, opiorphin has exhibited a prolonged analgesic profile (the behavioral response is recorded and analyzed in kinetics for 60 min) with a significant dose-dependent effect (p ≤ 0.002, Kruskal-Wallis variance analysis, n=8 rats/group) and a maximum anti-nociceptive potency at 1-2 mg/kg i.v. (Mann-Whitney test p = 0.036-0.003 vs. vehicle).

The following step was to define the specificity of the antinociceptive action mechanisms of opiorphin and notably the implication of endogenous opioid receptors. The antagonistic effect of the following ligands was analyzed: an opioid antagonist with a wide spectrum (Naloxone, 3 mg/kg) and selective antagonists of the opioid µ-receptors (CTOP, 0.8 mg/kg), δ-receptors (Naltrindole, 10 mg/kg) and κ-receptors (Nor-Binaltorphimine, 5 mg/kg), respectively.

**Table 1**

| Treatment | Vehicle | opiophin | Naltrindole + opiorphin | Nor-Binaltorphimine + opiorphin | CTAP + opiorphin |
|---|---|---|---|---|---|
| Variable | (n = 8) | (n = 8) | (n = 8) | (n = 8) | (n = 8) |
| Number of spasms | 287±31 | 149±28 | 176±29 | 169±26 | 285±36 |
| H(ddl=4)= 13.12; p<0.01 | | U = 8 | U = 10 | U = 9 | U = 32 |
| Mann-Whitney test Significance (vs.vehicle) | | p = 0.01 | p = 0.02 | p = 0.02 | p = 1.00 |
| Mann-Whitney test (vs. opiorphin 1 mg/kg) | | | U = 26.5 | U = 29 | U = 11 |
| Significance | | | p = 0.56 | p = 0.75 | p = 0.02 |

It was shown that the analgesic effect of opiorphin in the "formalin test" is abolished in the presence of Naloxone or CTPA, a specific antagonist of µ-opioid receptors (Table 1 above). This result demonstrates that the antinociceptive action of opiorphin is mediated by the endogenous opioid pathways which are dependent on the opioid receptors and requires specific activation of the receptors of the µ-opioid subtype. These receptors are involved in the transmission of physiological signals involved in the negative retrocontrol of the pain transmission (enkephalins, β-endorphin and endomorphins) and in the morphine action.

Thus, it was demonstrated that opiorphin exerts a potent antinociceptive activity at 1 mg/kg via activation of endogenous opioidergic pathways, in two analytical models of pain behavioral response in rats, the "Pin pain Test" (acute mechanical pain) and "Formalin Test" (chronic chemical inflammatory pain). On the other hand, opiorphin at 1 mg/kg i.v. is capable of inducing in both of these tests the maximum analgesic effect induced by morphine at 2-6 mg/kg i.p., the most active analgesic molecule in the treatment of severe and chronic pain in humans.

Moreover, it was seen with a colic inflammation model in rats that opiorphin at 1 mg/kg i.v. does not protect the colic wall from inflammation induced by intrarectal administration of TNBS, unlike ibuprofen. Opiorphin therefore does not exert any significant intestinal anti-inflammatory effect in this test. The anti-pain effect of opiorphin at 1 mg/kg in the "Formalin Test" therefore does not seem to be associated with anti-inflammatory activity of the peptide.

### Example 3: Effect of opiorphin on pathways for controlling emotions: antidepressant potential

### • The behavioral despair test (Forced Swimming Test)

The effects of opiorphin on the despair or resignation behavior were investigated in adult male rats. The test includes a pre-test session of 15 minutes and a test session of 5 minutes, after 24 hours. During the pre-test session, the behavior of the rat is recorded during the first 5 minutes. At the end of the pre-test, the rat is removed from the water, delicately dried, treated and then put back in his dwelling cage. During the test, the rat is again deposited in water and its behavior is recorded for 5 minutes.

Opiorphin was administrated via an intravenous route (i.v.) immediately after the pre-test session, and 300 and 15 minutes before the test session with three doses (0.5, 1 or 2 mg/kg). The reference substance was 8-OH-DOAT (0.5 mg/kg) which is a 5-HT1 a agonist or imipramine (20 mg/kg), which is an inhibitor of serotonin reuptake.

**Table 2**

| Group | Vehicle | Opiorphin 0.5 mg/kg | Opiorphin 1 mg/kg | Opiorphin 2 mg/kg | 8-OH-DPAT 0.5 mg/kg |
|---|---|---|---|---|---|
| Pre-test Kruskal-Wallis Test H(ddl4)= 1.828 | | | | | |
| P = 0.767 | | | | | |
| Test Kruskal-Wallis Test H(ddl4)= 20.046 | | | | | |
| P = 0.0005 | | | | | |
| Wilcoxon's Test (Test vs. Re-test) | z = 2.24 | z = 1.05 | z = 2.524 | z = 2.313 | z = 2.524 |
| | p = 0.025 | p = 0.293 | p = 0.012 | p = 0.021 | p = 0.012 |

Kruskal-Wallis variance analysis has shown that the immobility time of rats of the different groups during the pre-test is not significantly different, while significant heterogeneity was observed for these same groups after treatment (Table 2 above).

Mann-Whitney's test has shown that the immobility time of rats treated with 1 and 2 mg/kg opiorphin and 8-OH-DPAT is significantly less than that of the rats from the control group, whereas the immobility time of the rats of the group treated with 0.5 mg/kg opiorphin tends to be less than that of the rats from the control group (Fig. 1).

The Wilcoxon test has shown that the immobility time in water of control rats significantly increases between both Test vs. Pre-test sessions. Indeed, the control rats resign themselves during the test, since they have retained that they were soon to be taken out of the water. Unlike the control rats, the immobility time of the rats of the groups treated with 1 and 2 mg/kg opiorphin and 8-OH-DPAT significantly decreases between the pre-test and the test. This indicates that the rats treated with opiorphin do not resign themselves and therefore opiorphin has an antiresignation (antidepressant) effect. Further, the immobility time of the rats of the groups treated with 1 and 2 mg/kg opiorphin and 8-OH-DPAT significantly decreases relatively to that of the control group. This indicates that opiorphin not only exerts an antiresignation effect, but also a psychostimulant effect.

As a conclusion, opiorphin, administered intravenously induces a protective dose-dependent effect against resignation and thus exerts maximum antidepressant potency at 1 mg/kg in the behavioral despair test in adult male rats.

In order to exclude a possible amnesia or hyperactivity effect of opiorphin, which may also explain absence of resignation in treated rats, two additional behavioral tests were carried out in order to verify the specificity of the antidepressant effect of opiorphin.

### • Passive Avoidance Test

With the passive avoidance test in rats it is possible to evaluate the degree of memorization of the animal during the application of an aversive stimulus from which it cannot escape. The device consists of two compartments separated by an open partition allowing the rat to pass from compartment to the other. The first compartment where the rat is deposited at the beginning of the test is illuminated and communicates through a sash door with a dark compartment and the floor of which includes an electrified grid. The passive avoidance test includes a pre-test session where the animals receive an electric shock (2 mA, 2s) when it enters the dark compartment, and a 3 minute test session after 24 hours, where the behavior of the animal is analyzed for its long term retention of the learned task (the grid of the dark compartment is no longer electrified in the test). Opiorphin was administered (1 mg/kg i.v.) immediately after the session of pre-test. With this test it was possible to evaluate the amnesia effect of opiorphin. The table below shows the latency time for entering the dark compartment (average ± SEM, n=8 rats/group).

**Table 3**

| Group | Vehicle | Opiorphin 1 mg/kg | Mann-Whitney U test |
|---|---|---|---|
| | (n=8) | (n=8) | |
| Pre-test | 23.1 ± 4.7 | 24.6 ± 6.6 | U=31 p=0.91 |
| Test | 171.3 ±6.6 | 158.5 ±20.5 | U=32 p=0.99 |
| Wilcoxon's test | z = 2.52 | z = 2.53 | |
| | p = 0.012 | p = 0.012 | |

The Mann-Whitney test has shown that the latency time of the rats from both groups, the control and opiorphin groups, for entering the dark compartment during the two pre-test and test sessions, is not significantly different. The Wilcoxon test has shown that the latency time of rats for each of the two groups during the test is significantly higher (x 6-7) than the one obtained during the pre-test. This indicates that the animals have retained the learned task in the long term.

Opiorphin, at the dose of 1 mg/kg i.v. therefore does not show any amnesia effect in the passive avoidance test.

### • The locomotor activity test

The device consists of two compartments separated by an open partition allowing the rat to pass from one compartment to the other. The measured variables are the number of upright standing movements (vertical activity) and the number of passages into the two compartments (horizontal activity) during a period of 3 min. The reference molecule is imipramine (20 mg/kg).

**Table 4**

| Group | Vehicle | Imipramine 20 mg/kg | Opiorphin 1 mg/kg |
|---|---|---|---|
| | (n = 8) | (n=8) | (n = 8) |
| Average ± SEM | 30.13 ± 2.56 | 18.75 ± 1.91 | 35.25 ± 2.84 |
| Kruskal-Wallis test: (H(adf)=15.6; p=0.004) | | U=5 | U=21 |
| Mann-Whitney test (vs. vehicle) Significance | | p=0.008 | p=0.25 |

As compared with the control groups, opiorphin at the dose of 1 mg/kg i.v. neither induce any sedative effect, unlike imipramine (20 mg/kg i.p.), nor any significant hyperactivity effect, in the locomotor activity test in rats.

Thus, in an analytical model of behavioral despair, it was shown that in male rats, opiorphin exerts a specific anti-depressant effect at 1 mg/kg i.v. in addition to a psychostimulant effect.

The following step was to test the specificity of its action mechanisms and notably the implication of endogenous opioid receptors in the forced swimming test. The table below shows the effect of the treatment in the forced swimming test, by measuring the immobility time during the pre-test and the test sessions (s, n=8/group; average ± SEM)

**Table 5**

| Group | Vehicle (n=8) | | Imipramine 20 mg/kg | Opiorphin 1 mg/kg | Naltrindole Opiorphin 10-1 mg/kg | CTAP-Opiorphin 1-1 mg/kg |
|---|---|---|---|---|---|---|
| | (n = 8) | | (n = 7)* | (n = 8) | (n = 8) | (n = 8) |
| Pre-test | 62.9 ±11.8 | | 97.4±7.8 | 93.6±15.5 | 48.8±6.0 | 71.9±8.9 |
| Test | 128.3 ±21.1 | | 63.9±10.6 | 53.6±11.4 | 86.0±15.6 | 57.6±8.7 |
| KWT: (H(4df)=11.64;P=0.02) | | | U=6 | U=10 | U=16 | U=6 |
| Mann-Whitney test (vs. Vehicle in the test) Significance | | | p=0.011 | p=0.021 | p=0.093 | p=0.006 |
| Wilcoxon test (pre-test vs. test) | | Z=2.52 | z = 2.36 | z = 2.38 | z = 2.52 | z = 0.98 |
| Significance | | p=0.012 | p = 0.018 | p = 0.017 | p = 0.012 | p = 0.33 |

This second series of tests in the behavioral despair model in rats confirmed that opiorphin at 1 mg/kg i.v. induces an antidepressant effect in addition to a psychostimulant effect. This effect is comparable with the one exerted by the reference substance imipramine at 20 mg/kg i.p. The effects of opiorphin are abolished in the presence of Naltrindole, a specific antagonist of δ-opioid receptors (Table 5 above). This indicates that opiorphin's action of the antidepressant and psychostimulant type is mediated by endogenous opioid pathways and requires specific activation of the opioid receptors of the δ subtype.

### Example 4: Effect of opiorphin on pathways controlling emotion: anxiolytic potential

### • The conditioned defensive burying test

The anxiolytic effects of opiorphin, administered intravenously at the dose of 1 mg/kg were evaluated in the conditioned defensive burying test in Wistar male rats.

In order to avoid false positives, given that opiorphin exerts an analgesic effect, the test took place over two sessions: a session for selecting anxious rats followed by a test session under treatment. The conditioned defensive burying test for selecting experimental rats takes place in the first hours of the darkness phase, a phase where rats are most active. Each rat is deposited in the experimental device on the side opposite to the probe and a single electric shock of low intensity (2 mA), is delivered to the animal at the moment when it lays a front paw for the first time on the probe, and then its behavior is observed for 3 minutes. The sawdust is changed and leveled to a uniform height of 5 cm before the passage of each rat. Sixteen rats having expressed at least 25 seconds of burying the probe were selected.

The next day, the rat is treated 15 minutes before the test and deposited in the experimental device in the portion opposite to the probe. In this session, the rat does not receive an electric shock, the sole view of the probe triggers anxiety in rats. The behaviors of the rats are recorded for 5 minutes. The analyzed variables are the burying time of the probe, the number of stretchings in the direction of the probe, the number of approaches facing the probe, and the number of escapes with regard to the probe. These different variables are used for calculating an overall anxiety score for each rat. The table below shows the results obtained concerning the effects of opiorphin (1 mg/kg, i.v.) on the overall anxiety score in the conditioned burying test.

**Table 6**

| Groups | Vehicle | Opiorphin |
|---|---|---|
| | | 1 mg/kg |
| | (n = 8) | (n = 8) |
| Average ± SEM | 25.06 ± 2.94 | 25.94 ± 3.74 |
| Mann-Whitney test | U = 29.50 | |
| Significance | p = 0.79 | |

On the base of the overall anxiety score, opiorphin, at the dose of 1 mg/kg i.v. did not show any anxiolytic activity relatively to the control in the conditioned burying test in Wistar male rats. As regards upright standing movements, the rats treated with opiorphin showed a tendency of expressing less upright standing movements than the control rats (p=0.11 vs. vehicle, n=8 rats/group).

### Example 5: Effect of opiorphin in the pharmaco-dependency behavior: addiction type potential

### • Conditioned place preference test

The device consists of two compartments separated by an open sash door during the habituation phase and the test, allowing free passage of the rat from one compartment to the other. One compartment is illuminated and the floor includes a metal grid. This compartment is a non-preferential aversive compartment, and the time spent in this compartment is the measured variable. During the conditioning period of 10 days (45 min, closed sash door) the aversive compartment is associated with the administration of the product to be tested (control and drugs) while the preferential compartment (dark, bedding material) is associated with the control (vehicle). The test takes place over three phases: the habituation or pre-test phase, the conditioning phase and the final teaching phase. opiorphin was administered at the analgesic effective dose of 1 mg/kg i.v. and morphine was used as a reference substance administered at the dose of 2 mg/kg i.v.

Kruskal-Wallis variance analysis applied on the basis of the time spent in the non-preferential compartment, respectively during phase 1 (pre-test on day 0) and during phase 3 (test on day 11), indicated that the three groups do not significantly differ after the habituation phase on day 0 (H(2df)=1.21; p=0.55), while these same groups are significantly different during the test on day 11 after the conditioned treatment phase (H(2df)=7.91; p=0.02). In the latter case, the Mann-Whitney test has shown a significant difference between rats treated with morphine and those treated with the vehicle (U=1; p=0.001). On the other hand there was no significant difference between the rats conditioned with opiorphin and the controls conditioned with the vehicle (U=31; p=0.92).

After chronic treatment with analgesic doses of opiorphin, the rats did not develop significant preference for the compartment initially established as non-preferential. Indeed, these rats spent as much time in the non-preferential compartment as during the initial pre-test phase (Wilcoxon's test: z=1.54; p=0.12). On the other hand, after chronic treatment with analgesic doses of morphine, the rats developed a significant preferential location for the compartment initially established as non-preferential during the pre-test (z=2.52; p=0.012).

At effective analgesic doses in the "Formalin Test", opiorphin therefore does not cause significant pharmaco-dependence, unlike morphine. Repeated administration of opiorphin at the dose of 1 mg/kg does not induce significant adverse effect of addictive type in the conditioned place preference test.

### Example 6: No occurrence of opirophin antinociceptive tolerance in the Tail-flick test

### • Material and Methods

Male Wistar rats (Harlan, France) weighing 250 to 280 g at the beginning of the experiment were used in this study. After 7-day acclimatization period, they were weighed and randomly housed according to the treatment groups in a room with a 12 h alternating light/dark cycle (21:00 p.m./9:00 a.m.) and controlled temperature (21±1°C) and hygrometry (50±5%). Food and water were available ad libidum. They were experimentally only tested once. Behavioral tests, care and euthanasia of study animals were in accordance with guidelines of the European Communities Directive 86/609/EEC and the ASAB Ethical Committee for the use of laboratory animals in behavioral research 71:245-53.

Opiorphin (Genosphere Laboratory, France) was dissolved in vehicle solution (55% of PBS 100mM - 45% of Acetic acid 0.01 N) and systemically injected 5 to 15 min prior the behavioral tests at doses ranging from 0.5 to 2 mg/kg body weight. Morphine HCl purchased from Francopia (France) was dissolved in saline (0.9% sodium chloride in distilled water) and injected via the i.v. route 15 min before the behavioral test at 1-2 mg/kg dose. Naloxone (a centrally and peripherally acting opioid antagonist) was purchased from Sigma Chemical (France) and dissolved in saline and subcutaneously (s.c.) administered at 3 mg/kg, 15 min before Opiorphin administration. Naltrindole (δ-opioid antagonist), Nor-Binaltorphimine (κ-opioid antagonist) and CTAP (µ-opioid antagonist) were purchased from Sigma Chemical (France), dissolved in saline and administered at 10 mg/kg i.p., 20 min; 5 mg/kg i.p., 3h and 0.8 mg/kg i.v., 25 min before tests, respectively. All drugs were administered in a volume of 1 ml/kg body weight.

The tail-flick test evaluating the time required to respond to acute thermal stimulus preferentially reflects nociceptive spinal reflex. A standardized tail-flick apparatus (Harvard Apparatus LTD, Edenbridge, England) with a radiant heat source connected to an automated tail-flick analgesymeter was used. Rats were accustomed to the situation of contention for two 2-min sessions, the day prior the test. On experimental day, they were gently restrained by hand so the radiant heat source was focused onto the distal dorsal surface of the tail. The previously adjusted intensity of the thermal stimulus was set at 30% to obtain a basal tail-flick latency ≤2-3 sec. Under these experimental conditions and in reference to morphine, a 5 sec cut-off time was established to prevent tissue damage. For each behavioral test rat was used as its own control and 2 consecutive measurements separated by 30 s intervals were carried out before Opiorphin, morphine or vehicle injection to assess for baseline tail-flick latency. On testing day, rats received an intravenous administration of one of the following freshly prepared solutions: vehicle solution, 1 mg/kg morphine or 2 mg/kg Opiorphin. For tolerance induction rats received daily intravenous administration of freshly prepared solutions during seven consecutive days. Results were expressed as means of tail-flick latency ± SEM for n=6 rats.

The results were expressed as mean ± SEM. The significance of differences between groups was evaluated using the Kruskal-Wallis one-way analysis of variance by ranks (KWT, a non-parametric method) for comparison between several independent variables across the experimental conditions. When a significant difference among the treatments was obtained, the Mann-Whitney post hoc test (MWT) was applied to define which group contributed to these differences by comparing each treated group to the control one (vehicle or baseline values). The non-parametric Wilcoxon matched pairs test (WT) was used to compare two paired variables with repeated measures in each treatment group. For all statistical evaluations, the level of significance was set at P<0.05. All statistical analyses were carried out using the computer software StatView®5 statistical package (SAS, Institute, Inc., USA).

### • Human Opiorphin exhibits an antinociceptive effect in the Tail-flick test

The tail-flick test measures the time required to respond to a painful radiating thermal stimulus. Tail withdrawal latency determined the thermal nociceptive threshold, which is functionally related to pain responsiveness to noxious stimulus. Drug-induced attenuation of the tail-flick response provides preliminary evidence for pain antinociception, which is preferentially integrated at spinal level. The aim of the study was to assess the potency and the duration of Opiorphin analgesic effect compared to morphine, using the rat tail-flick test t.

For this study, a systemic dose of 2 mg/kg Opiorphin was chosen in order to investigate the maximal response in term of duration of action in the rat tail-flick test. This dose was evaluated according to preliminary data showing that Opiorphin induced significant dose-dependent antinociceptive responses with maximal effect at 2 mg/kg. Tail-flick latencies were evaluated for 4 different time points: 5, 15, 25 and 60 min after Opiorphin or vehicle administration in comparison with morphine (1 mg/kg, i.v.).

Figure 2 shows the mean tail flick latency in function of time under Opiorphin or Morphine or vehicle-treatment conditions (n=6 rats per group). The pre-injection baseline values did not significantly differ between the 3 groups (P=0.54 by Kruskal-Wallis test). Similarly, no significant difference appeared between the mean tail flick latency of the 3 groups at 5 min-post-injection (P=0.34). On the other side, the one-way Kruskal-Wallis analysis revealed a significant effect of treatments 15, 25 and 60 minutes post-injection (P=0.005, P=0.005 and P=0.02, respectively). Subsequent individual means comparisons of tail flick latencies between Opiorphin- or morphine-treated rats and control rats treated with vehicle indicated that the time latency significantly increased 15 and 25 minutes after administration of both Opiorphin and morphine: from 2.57±0.10 and 2.51±0.06 sec for vehicle to 3.56±0.32 sec and 3.29±0.15 sec, P=0.01 and P=0.008 vs vehicle, respectively for morphine; and to 3.12±0.07 sec and 3.19±0.07 sec, P=0.005 and P=0.004 vs vehicle, respectively for Opiorphin by Mann-Whitney test (MWT). It was still significant 60 min later for morphine (P=0.01 vs vehicle) while tended to be higher for Opiorphin (P=0.08 vs vehicle). Opiorphin-treated rats did not significantly differ in response latencies from those of morphine-treated ones at 15, 25 and 60 minutes after treatment (P=0.20, P=0.34 and P=0.26 vs morphine by MWT, respectively).

In addition, comparison with respective pre-injection baseline values showed that the tail flick latency significantly increased at 5, 15 and 25 minutes after treatment with morphine (P=0.05, P=0.03 and P=0.05 by Wilcoxon test WT, respectively). On the other hand, treatment with Opiorphin induced significant increase in the response latency at 15 and 25 min post-injection time-points (P=0.03 and P=0.03 by WT, respectively). Interestingly, comparison to corresponding baseline response for the control group revealed a significant time effect on thermal pain threshold following repeated exposure to the test, i.e., a progressive decrease in the time latency of tail withdrawal. The diminution of pain threshold to a repetitive stimulus may reflect painful sensitization or learning to the stimulus leading to a facilitation of the nociceptive response in these animals. The enhanced nociceptive response to thermal stimulus following repeated measures was totally reversed in rats treated with Opiorphin similarly to morphine, confirming its potent analgesic potency on thermal acute pain.

In the rat tail-flick paradigm, the maximum analgesic effect of Opiorphin (2 mg/kg i.v.) occurred 15 to 25 min after i.v. administration and was of the same range of magnitude, in terms of amplitude and duration of action, than that of morphine (1 mg/kg i.v.). This demonstrates that Opiorphin inhibits thermal injury-evoked acute pain behavior, which is controlled by spinal enkephalinergic pathways.

### • Comparison of occurrence of antinociceptive tolerance between Human Opiorphin and morphine chronic treatment in the Tail-flick test

The aim of the experiment was to investigate, using the tail-flick test, the potential emergence of Opiorphin-induced antinociceptive tolerance. For tolerance induction rats received daily intravenous administration of Opiorphin (2 mg/kg), morphine (1 mg/kg) or vehicle during seven consecutive days. Then the tail flick latency was measured at peak effect time, i.e., 15 min and 25 min after the challenge dose.

Comparing pre-injection baseline values, no significant difference between the mean tail flick latency of the 3 groups was observed (P=0.54 on day 1 and P=0.14 on day 7 by KWT, n=6 rats/group). While, the Kruskal-Wallis analysis revealed a significant treatment effect in tail flick latency among the 3 groups (P=0.005 on day 1; P=0.003 on day 7 at 15 and 25-min post-injection time-points, respectively). When comparing time-response profile between day 7 and day 1, the tail-flick latencies remained stable (P≥0.99 by Wilcoxon test, WT) for vehicle-treated group, tended to decrease for morphine-treated group (P=0.06) and tended to increase for Opiorphin-treated group (P=0.06 by WT) after daily repeated treatment (Figure 3).

Compared to vehicle treatment group a significant increase in response latencies was observed following a single acute administration of morphine or Opiorphin on first day (P=0.01 or P=0.01, respectively by MWT post-hoc comparisons at both 15 and 25-min post-injection time-points). After 7 consecutive days chronic treatment, morphine and Opiorphin were able to still induce a significant increase in the tail-flick response 15 and 25 min post-injection when compared to vehicle chronic treatment (P=0.02 and P=0.004, respectively). However, the morphine-induced increase tended to be lower than the response induced by Opiorphin (P=0.08 by MWT at 15 min post-injection time-point).

On the other hand, comparison with respective pre-injection baseline values showed that after 7 day-chronic treatments, the tail flick latency remained stable 15 minutes after the challenge dose of morphine and significantly decreased at 25 min post-dose (P=0.69 and P=0.03 by WT) reflecting an absence of morphine antinociceptive potency following chronic treatment. In the same experimental conditions, the challenge dose of Opiorphin significantly increased the tail-flick response at 15-min post-injection (P=0.03 by WT) whereas it seems to have no significant effect at 25-min post-injection (P=0.67). Thus, the analgesic intensity of Opiorphin was unaltered after chronic treatment while a decrease in the duration of the antinociceptive response seems to appear.

In conclusion, unlike morphine, chronic systemic administration of Opiorphin (2 mg/kg, i.v.) administered once daily for 7 days does not induce the development of tolerance to maximal antinociceptive effect in tail-flick test in rats.

### Example 7: Conclusion

The experimental data above therefore demonstrate that opiorphin exerts at 1 mg/kg i.v. a potent and tonic antinociceptive activity via activation of opioidergic pathways dependent on endogenous µ-opioid receptors. This result was confirmed in three analytical models of the acute behavioral response in rats, the "Pin pain Test" (acute mechanical pain), the "Formalin Test" (chronic chemical inflammatory pain) and the "tail-flick test" (acute thermal pain). Opiorphin is capable of inducing the maximum analgesic effect induced by morphine (2-6 mg/kg i.p.) in these tests, without inducing any of the major adverse effects of morphine, i.e. the anti-peristaltic effect, the pharmacodependence effect and the tolerance effect (Rougeot C, Proceedings of the 4th International Peptide Symposium; J. Wilce (Editor) on behalf of the Australian Peptide Society, 2007). Further, the specific (non-anti-inflammatory) and pronounced analgesic effect of opiorphin in the chronic chemical pain test is in favor of potential antinociceptive action of opiorphin in severe neuropathic pain.

Further, in an analytical model of behavioral despair in male rats, the inventors have shown that opiorphin exerts a specific antidepressant effect at 1 mg/kg i.v. in addition to a psychostimulant effect, via activation of the opioidergic pathways dependent on endogenous δ-opioid receptors. The effect of opiorphin in this test is specific since the rats treated with the same dose do not develop any response of the amnesic, sedative or hyperactive type in various suitable behavioral tests.

The anti-pain, antidepressant and psychostimulant effects of opiorphin are dependent on the activation of the endogenous µ- and δ-opioid receptors which transmit the action of the endogenous enkephalins released in response to the stimulus (pain, stress, emotions...).

Moreover, opiorphin at the dose of 1 mg/kg i.v. did not show any anxiolytic activity during the conditioned defensive burying test in male rats.

Finally, in the conditioned place preference test and at effective analgesic doses in the formalin test, opiorphin does not cause any significant pharmaco-dependence. Repeated administration of 1 mg/kg of opiorphin does not induce any significant adverse effect of addictive and/or tolerance type, unlike morphine.

## Claims

1. A peptide for use as an analgesic agent for chronic treatment of pain, wherein said peptide consists of the sequence QRFSR (SEQ ID NO: 2), or Glp-RFSR and wherein said chronic treatment does not induce pharmacodependence.

2. The peptide for the use according to claim 1, wherein said chronic treatment does not induce tolerance.

3. The peptide for the use according to claim 2 wherein said peptide is QRFSR (SEQ ID NO: 2) administered at 2 mg/kg i.v. once daily for 7 days.

4. The peptide for the use according to claim 1, wherein said chronic treatment comprises repeated administration of the peptide.

5. The peptide for the use according to any of claim 1 or 4, wherein said peptide is QRFSR (SEQ ID NO: 2) administered at 1 mg/kg i.v. once daily for 11 days

6. The peptide for the use according to claim 1 wherein said peptide is administered to an adult at a dose less than or equal to 75 mg per day.

7. The peptide for the use according to claim 1, wherein said peptide is administered to a child at a dose less than or equal to 25 mg per day.

8. The peptide for the use according to claim 6 or 7, wherein said peptide is administered three times daily.


SEQUENCE LISTING

<110>  INSTITUT PASTEUR

<120>  OPIORPHIN FOR USE AS ANALGESIC AGENT

<130>  BET 15P2763

<150>  FR 08 53400
<151>  2008-05-26

<160>  6

<170>  PatentIn version 3.5

<210>  1
<211>  6
<212>  PRT
<213>  Artificial

<220>
<223>  peptide


<220>
<221>  misc_feature
<222>  (1)..(2)
<223>  Xaa can be any naturally occurring amino acid

<400>  1

Xaa Xaa Arg Phe Ser Arg
1               5


<210>  2
<211>  5
<212>  PRT
<213>  Homo sapiens

<400>  2

Gln Arg Phe Ser Arg
1               5


<210>  3
<211>  6
<212>  PRT
<213>  Artificial

<220>
<223>  peptide

<400>  3

Tyr Gln Arg Phe Ser Arg
1               5


<210>  4
<211>  6
<212>  PRT

<213>  Artificial

<220>
<223>  peptide

<400>  4

Cys Gln Arg Phe Ser Arg
1               5


<210>  5
<211>  201
<212>  PRT
<213>  Homo sapiens

<400>  5

Met Lys Leu Thr Phe Phe Leu Gly Leu Leu Ala Leu Ile Ser Cys Phe
1               5                   10                  15

Thr Pro Ser Glu Ser Gln Arg Phe Ser Arg Arg Pro Tyr Leu Pro Gly
            20                  25                  30

Gln Leu Pro Pro Pro Pro Leu Tyr Arg Pro Arg Trp Val Pro Pro Ser
        35                  40                  45

Pro Pro Pro Pro Tyr Asp Ser Arg Leu Asn Ser Pro Leu Ser Leu Pro
    50                  55                  60

Phe Val Pro Gly Arg Val Pro Pro Ser Ser Phe Ser Arg Phe Ser Gln
65                  70                  75                  80

Ala Val Ile Leu Ser Gln Leu Phe Pro Leu Glu Ser Ile Arg Gln Pro
                85                  90                  95

Arg Leu Phe Pro Gly Tyr Pro Asn Leu His Phe Pro Leu Arg Pro Tyr
            100                 105                 110

Tyr Val Gly Pro Ile Arg Ile Leu Lys Pro Pro Phe Pro Pro Ile Pro
        115                 120                 125

Phe Phe Leu Ala Ile Tyr Leu Pro Ile Ser Asn Pro Glu Pro Gln Ile
    130                 135                 140

Asn Ile Thr Thr Ala Asp Thr Thr Ile Thr Thr Asn Pro Pro Thr Thr
145                 150                 155                 160

Ala Thr Ala Thr Thr Arg His Phe His Lys Thr His Asn Asp Asp Gln
                165                 170                 175

Leu Leu Asn Ser Thr Tyr Leu Phe Asn Thr Arg Ala Cys His Leu His

            180                 185                 190

Ile Ser Ser Asn Pro Arg Ser Ile Tyr
        195                 200

<210> 6
<211> 5
<212> PRT
<213> Rattus norvegicus

<400> 6

Gln His Asn Pro Arg
1               5